# EUROPÄISCHE PATENTSCHRIFT

(11) **EP 1 156 767 B1**
(45) Veröffentlichungstag und Bekanntmachung des Hinweises auf die Patenterteilung: **27.10.2004**
(21) Anmeldenummer: 00909084.6
(22) Anmeldetag: 19.01.2000
(51) Int. Cl.: A61F 13/62, A44B 18/00

(54) **KLETTVERBINDUNG FÜR FLÄCHIGE GEBILDE**
HOOK AND LOOP FASTENER FOR FLAT MATERIALS
FERMETURE ADHESIVE POUR PRODUITS EN NAPPE

(30) Priorität: 25.01.1999 DE 19902762
(43) Veröffentlichungstag der Anmeldung: 28.11.2001
(73) Patentinhaber: Carl Freudenberg KG, 69469 Weinheim (DE)
(72) Erfinder: JÖST, Manfred, D-69502 Hemsbach (DE); GROITZSCH, Dieter, D-69493 Hirschberg (DE); SCHAUT, Gerhard, D-69502 Hemsbach (DE)
(86) Internationale Anmeldenummer: PCT/EP2000/000371
(87) Internationale Veröffentlichungsnummer: WO 2000/042964

(56) Entgegenhaltungen:
- EP-A- 0 258 015

## Beschreibung

Eine weitere Verbreitung haben heute sogenannte Klettverschlüsse gefunden, die auf sehr einfache und schnelle Weise eine Verbindung von verschiedenen flächigen Gebilden zulassen. Sowohl die Verbindung von aneinander anstoßenden Kanten als auch der Einsatz als Verschlußriemen oder dergleichen sind üblich. Die Klettverschlüsse bestehen generell aus zwei Bestandteilen und zwar dem Hakenteil aus extrudierten einfachhakigen, doppelhakigen oder pilzförmigen Erhebungen und einem Verhakungsteil mit schlingenförmiger Anordnung von Fasern oder Fäden für das Einhaken des Hakenteils. Man unterscheidet allgemein zwischen sehr stabilen Klettverschlüssen, beispielsweise an Schuhen, welche eine hohe Zahl von Schließ- und Öffnungsvorgängen aushalten müssen und solchen Klettverschlüssen, welche nur wenige Male geschlossen und geöffnet werden, beispielsweise an Wegwerfprodukten. Solche Wegwerfprodukte sind beispielsweise Klettverschlüsse an Hygieneprodukten, wie Kinderwindeln oder Erwachseneninkontinenz-Windeln.

### Stand der Technik

In der internationalen Patentanmeldung WO 95/17111 ist eine Klettverbindung für Babywindeln und Inkontinenz-Produkten dargestellt. Der Hakenteil besteht aus einer Basisschicht mit Hakengliedem. Das Verhakungsteil wird aus einer Verstärkungslage und einem Verschlingungsmaterial gebildet, wobei das Schlingenmaterial unterhalb der Verstärkungseinlage angeordnet ist und die Schlingen durch Nadeln des Schlingenmateriais durch das Verstärkungsmaterial hindurchgestochen werden. Ein solcher Klettverschluß ist von geringer Festigkeit, insbesondere dann, wenn als Schlingenmaterial eine Schicht aus Stapelfasern verwendet wird.

In der EP PA 0 765 616 wird der Verhakungsteil eines Klettverschlusses behandelt, bei dem ein mechanisch oder hydrodynamisch vemadelter Vliesstoff aus Bikomponenten-Bindefasern verwendet wird. Der Vliesstoff wird durch einen Walzenspalt geführt, bei dem eine Walze über dem Schmelzpunkt der Bindefaser und die andere deutlich unter dem Schmelzpunkt der Bindefaser aufgeheizt ist. Durch das gebildete Temperaturgefälle wird die eine Seite des Vliesstoffes zu einer folienartigen Fläche verschmolzen und die andere Seite bleibt an der Oberfläche ungebunden. Durch den Nadelvorgang entstehen auf der Faseroberfläche Auswölbungen mit einer Vielzahl von Schlingen, welche das Einhaken der Haken ermöglichen. Auch bei dieser Ausführungsform läßt die Festigkeit des Verschlusses zu wünschen übrig. Von Nachteil ist ebenfalls, daß auch hier wie schon bei der vorstehend behandelten Schrift zum Stand der Technik gekräuselte Fasern zum Einsatz kommen und eine einigermaßen vertretbare Klettverbindung nur dann erreicht werden kann, wenn die Schlingenschicht in sich selbst gut abgebunden ist und gegebenenfalls mit einer Trägerschicht ausgestattet ist.

In der EP 0 258 015 A 2 werden verschiedene Möglichkeiten für die Herstellung von Verhakungsteilen behandelt, dabei wird das Verhakungsteil aus einem Vlies, aus einem Nadelfilzl-vlies oder aus einem Gewirke und einer damit durch Verkleben verbundenen thermoplastischen Harzschicht gebildet. Die Festigkeit eines solchen Verhakungsteils wird durch den Klebevorgang und die Dicke der Harzschicht bestimmt. Da die Harzschicht sehr dünn ist (0,001 bis 0,002 inch, siehe Sp. 5, Z. 13 und 14) und der Klebevorgang schwer durchzuführen ist, ergeben sich unzureichende Festigkeiten.

### Darstellung der Erfindung

Der Erfindung liegt die Aufgabe zugrunde, einen Verhakungsteil für eine Klettverbindung zu schaffen, der einfach herzustellen ist und eine hohe Festigkeit aufweist. Die Lösung der gestellten Aufgabe wird erfindungsgemäß dadurch erreicht, daß das Verhakungsteil aus einem Vliesstoff-Verbund mit eingewirkten texturierten polymeren Fäden besteht, wobei der Vliesstoff-Verbund als Träger für die texturierten Fäden dient und die texturierten Fäden die Schlingen für die Haken des Hakenteils bilden. Das so gebildete Verhakungsteil zeigt äußerst günstige Eigenschaften und ist den bisher bekannten Hakenteilen aus Vliesstoffen mit Kräuselfasem als eigentlichem Verhakungsmedium in der Effektivität deutlich überlegen. Bei der Ausführungsform nach der Erfindung ist nicht der Vliesstoff beziehungsweise dessen Fasern der Angriffspunkt für die Verhakung, sondern der Vliesstoff dient lediglich als Träger für die eingeraschelten texturierten Fäden, welche die Verhakungsschlingen bilden.

Zur Verwirklichung der Erfindung ist es auch möglich, den Vliesstoff-Verbund aus ungekräuselten Fasern hoher Festigkeit zu bilden. Dabei werden vorzugsweise Endlos-Fasern eingesetzt.

Möglich ist auch der Einsatz von Mikrofasern, wenn eine besondere Weichheit des Vliesstoff-Verbundes erreicht werden soll.

Besonders günstig ist eine Ausführungsform, bei der der Vliesstoff-Verbund aus mindestens zwei Vliesstoff-Schichten gebildet ist. Hier sind verschiedene Variationen möglich.

Eine gute Lösung wird dann erreicht, wenn der Vliesstoff-Verbund aus einer Spinnvliesstoff/Meltblown -oder einer SpinnvUesstoff/Mettbtown/SpinnvHesstoff-Komposition besteht. Hier wird zwischen zwei Spinnvtiesstoffen aus Endlosfäden eine sehr lockere Zwischenschicht erreicht.

Die einzelnen Schichten können locker übereinander gelegt werden. Es ist aber auch möglich, den Vliesstoff-Verbund vor Einbringung der Fäden musterartig verteilt zu verschweißen. Hier kann die Punktverschweißtechnik oder auch die Ultraschallverschweißung eingesetzt werden. Eine weitere Möglichkeit besteht darin, den Vliesstoff-Verbund vor Einbringung der Fäden ganzflächig zu kalandrieren. In besonderen Fällen ist auch eine Verklebung der Fäden über bindemittelhaltige Vliesstoffe denkbar.

Neben den bevorzugten Spinnvliesstoffen sind generell auch Stapelfaservliesstoffe oder Naßvliesstoffe einsetzbar. Aus Festigkeits- und auch aus Kostengründen werden jedoch Spinnvliesstoffe bevorzugt.

Sofern der Vliesstoff-Verbund ganz oder teilweise aus Mikrofasern besteht, können diese durch bekannte Splittmethoden durch Hochdruck-, Wasserstrahl-, Heißwasser- oder Dampfbehandlung oder trockenmechanische Behandlung erzielt werden.

Möglich ist auch eine Verfestigung des Vliesstoff-Verbunds durch Hochdruckwasserstrahl. Dabei ist jedoch zu beachten, daß der Vliesstoff-Verbund nicht makroperforiert wird.

In einer Reihe von Anwendungsfällen ist es erwünscht, daß der Verschlußbereich des Klettverschlusses farbig angelegt ist. insbesondere dann wenn der Klettverschluß auf den Nutzgegenstand aufgeklebt wird und eine Klebefläche enthalten soll. Eine solche Fläche kann durch Abglätten erreicht werden. Dieses kann besonders einfach durchgeführt werden bei einer Meltblown-Schicht, die folienartig abgeglättet werden kann, indem sie beispielsweise über eine erhitzte Walze geführt wird.

Um eine gute Druck- bzw. Klebefläche zu erreichen, kann der Vliesstoff-Verbund auch mit einer Unterschicht aus einer Folie versehen sein, auf welche der Druck bzw. die Klebeschicht aufgebracht wird. Die Folie kann durch Extrusion mit der aus Vliesstoff bestehenden Oberschicht verbunden werden.

Für den Vliesstoff Verbund wird im allgemeinen ein Flächengewicht von 7 bis 70 g/qm vorzugsweise 10 bis 50 g/qm gewählt. Die texturierten Fäden selbst können aus gleichen oder unterschiedlichen homofil und/oder Bikomponenten Filamenten bestehen. Bevorzugt werden jedoch Fäden aus biologisch abbaubaren Polymeren verwendet, um die für Windeln gewünschte Abbaubarkeit zu erreichen.

Die texturierten Fäden weisen einen Titer von 20 bis 200 dtex vorzugsweise von 30 bis 120 dtex auf und werden als Kettfäden in den Vliesstoff-Verbund eingebracht. Dabei kann in Kettrichtung eine zickzack-musterförmige Verteilung vorgenommen werden. Nach dem Einnähen der Fäden und Entspannen.des fadenverstärkten Vliesstoff-Verbundes kann es zu einer Längenverkürzung der Ware von 0 bis 10 % kommen.

Die Anzahl der Kettfäden beträgt 3 bis 25 pro **2,54 cm** (pro inch), vorzugsweise 20 pro **2,54 cm** (pro inch). Dadurch wird eine ausreichende Schlingenzahl für das Verhaken gebildet Die Maschenzahl beträgt 2 bis 4 pro Zentimeter.

Der erfindungsgemäße Verhakungsteil für einen Klettverschluß wird vorzugsweise bei Hygiene-Einmalprodukten wie Kinder- oder Erwachsenenwindeln eingesetzt. Auch andere Wegwerfanwendungen sind denkbar, bei denen höchstens ein zehnfaches Schließen und Wiederöffnen des Klettverschlusses verlangt wird. Dieses ist beispielsweise bei nicht wiederverwendbaren OP-Kitteln oder Arbeitsschutzbekleidung der Fall.

Generell wird bei der Ausführung der Erfindung ein Vliesstoff Verbund eingesetzt, der aus mehreren Schichten besteht, wobei mindestens eine aus Mikrofasern aufgebaut ist Die Mikrofaser-Schicht ist zumindest einseitig mit normalem Spinnviiesstoff- oder Stape)faserv!iesstoff abgedeckt. Auf zumindest einer der Außenlage des Vliesstoff-Verbundes wird eine haftungsfreundliche Lage gewählt, die vor dem Einraschein der texturierten Fäden mit einem Mehrfarben-Druck versehen wird. Hierzu kann zusätzlich an der nichtbedruckten Seite ein Klebstoff aufgetragen werden, vorzugsweise ein Haftkleber.

In vielen Fällen ist es jedoch günstig, wenn der Vliesstoff-Verbund mit einer Folie versehen ist, welche monolithisch sein kann oder Mikroporen enthält Die monolithische Folie kann entsprechend ihres Polymeraufbaus wasserdampfdurchlässig sein oder aber auch eine Sperre gegen Wassserdampf darstellen. Vorzugsweise werden Polyoietinische Folien eingesetzt, die gegebenenfalls Haftvermittler oder Kiebrigkeitsmacher enthalten oder durch energiereiche Strahlung, gegebenenfalls im Beisein von radikalischen Monomeren haftungsfreundlicher für den Verklebungsprozeß gestaltet worden sind.

Die bevorzugte Herstellungsform des Vliesstoff-Verbunds sieht vor, daß der Vliesstoff-Verbund in gedehnter Form einen Raschelvorgang zur Einbringung der Kettfäden zugeführt wird. Die Vordehnung erfolgt bei linearer Ausrichtung der eingeraschelten Fäden vorzugsweise nur in dieser Richtung. Bei einer Zickzack-Führung der texturierten Fäden ist eine Vordehnung des elastischen Vliesstoffes in Längs- und Querrichtung oder nur in einer der beiden Richtungen möglich. Durch die nachträgliche Entspannung des Vliesstoff-Verbunds erhöht sich die Anzahl der Maschen pro Längeneinheit und die Höhe der Schlingen entsprechend der Längenverkürzung.

## Patentansprüche

1. Klettverbindung für flächige Gebilde, insbesondere für textile Produkte mit einem Hakenteil und einem Verhakungsteil, wobei das Vethakungsteil aus einem Träger aus einem Vliesstoff-Verbund mit in den Vliesstoff-Verbund eingewirkten texturierten polymeren Fäden für die Schlingen des Verhakungsteils besteht, **dadurch gekennzeichnet, dass** der Vliesstoff-Verbund aus ungekräuselten Endlos-Fasern hoher Festigkeit besteht und aus mindestens zwei Vliesstoff-Schichten gebildet ist.

2. Klettverbindung nach Anspruch 1, **dadurch gekennzeichnet, dass** der Vliesstoff-Verbund aus einer Spinnvtiesstoff/Mettbtown oder einer Spinnvliesstoff/MettbIown/Spinnvliesstoff Komposition besteht.

3. Klettverbindung nach Anspruch 2, **dadurch gekennzeichnet, dass** die Mettblown-Schicht vor Einbringung der Fäden folienartig abgeglättet ist.

4. Klettverbindung nach einem der Ansprüche 1 bis 3, **dadurch gekennzeichnet, dass** der Vliesstoff-Verbund vor Einbringung der Fäden musterartig verteilt verschweißt ist.

5. Klettverbindung nach einem der Ansprüche 1 bis 4, **dadurch gekennzeichnet, dass** der Vliesstoff-Verbund vor Einbringung der Fäden ganzflächig kalandriert ist.

6. Klettverbindung nach einem der Ansprüche 1 bis 5, **dadurch gekennzeichnet, dass** der Vliesstoff-Verbund eine Unterschicht aus einer Folie hat.

7. Klettverbindung nach Anspruch 6, **dadurch gekennzeichnet, dass** die Folie durch Extrusion mit der aus dem Vliesstoff-Verbund bestehenden Oberschicht verbunden ist.

8. Klettverbindung nach einem der Ansprüche 1 bis 7, **dadurch gekennzeichnet, dass** der Vliesstoff-Verbund ein Flächengewicht von 7 bis 70 g/qm, vorzugsweise 10 bis 50 g/qm hat.

9. Klettverbindung nach einem der Ansprüche 1 bis 8, **dadurch gekennzeichnet, dass** die Vliesstoffe und/oder die texturierten Fäden aus gleichen oder unterschiedlichen Homofil- und/oder Bikomponenten-Filamenten bestehen.

10. Klettverbindung nach einem der Ansprüche 1 bis 9, **dadurch gekennzeichnet, dass** der Vliesstoff-Verbund und die texturierten Fäden aus biologisch abbaubarem Polymer bestehen.

11. Klettverbindung nach einem der Ansprüche 1 bis 10, **dadurch gekennzeichnet, dass** die texturierten Fäden einen Titer (Fadenstärke) von 20 bis 200 dtex, vorzugsweise von 30 bis 120 dtex aufweisen.

12. Klettverbindung nach einem der Ansprüche 1 bis 12, **dadurch gekennzeichnet, dass** die texturierten Fäden als Kettfäden in den Vliesstoff-Verbund eingebracht sind.

13. Klettverbindung nach einem der Ansprüche 1 bis 12, **dadurch gekennzeichnet, dass** die Anzahl der Kettfäden (Kettdichie) 3 bis 25 pro 2,54 cm, vorzugsweise 20 pro 2,54 cm beträgt.

14. Klettverbindung nach einem der Ansprüche 1 bis 13, **dadurch gekennzeichnet, dass** die Maschenzahl 2 bis 4 pro cm beträgt.

15. Verfahren zur Herstellung einer Klettverbindung nach einem der Ansprüche 1 bis 14, **dadurch gekennzeichnet, dass** der Vliesstoff-Verbund einem Raschelvorgang zur Einbringung der Kettfäden in gedehnter Form zugeführt wird.

## Claims

1. A hook and loop fastener for sheetlike structures, especially for textile products, which comprises a hook portion and a hook-engaging portion, the hook-engaging portion consisting of a bonded nonwoven fabric assembly backing having, loop-formingly knitted into the bonded nonwoven fabric assembly, textured polymeric threads for the loops of the hook-engaging portion, **characterized in that** the bonded nonwoven fabric assembly consists of uncrimped continuous filament fibres of high strength and is formed of at least two layers of nonwoven fabric.

2. A hook and loop fastener according to claim 1, **characterized in that** the bonded nonwoven fabric assembly consists of a spunbond-meltblown composite or of a spunbond-meltblown-spunbond composite.

3. A hook and loop fastener according to claim 2, **characterized in that** the meltblown layer is smoothed off to be film-like before the threads are introduced.

4. A hook and loop fastener according to any one of claims 1 to 3, **characterized in that** the bonded nonwoven fabric assembly is welded together in a distributed pattern before the threads are introduced.

5. A hook and loop fastener according to any one of claims 1 to 4, **characterized in that** the bonded nonwoven fabric assembly is uniformly calendered before the threads are introduced.

6. A hook and loop fastener according to any one of claims 1 to 5, **characterized in that** the bonded nonwoven fabric assembly has an underlayer, the underlayer consisting of a film.

7. A hook and loop fastener according to claim 6, **characterized in that** the film is bonded by extrusion to the overlayer consisting of the bonded nonwoven fabric assembly.

8. A hook and loop fastener according to any one of claims 1 to 7, **characterized in that** the bonded nonwoven fabric assembly has a basis weight in the range from 7 to 70 g/square metre and preferably in the range from 10 to 50 g/square metre.

9. A hook and loop fastener according to any one of claims 1 to 8, **characterized in that** the nonwoven fabrics and/or the textured threads consist of identical or different homofil and/or bicomponent filaments.

10. A hook and loop fastener according to any one of claims 1 to 9, **characterized in that** the bonded nonwoven fabric assembly and the textured threads consist of biodegradable polymer.

11. A hook and loop fastener according to any one of claims 1 to 10, **characterized in that** the textured threads have a linear density in the range from 20 to 200 dtex and preferably in the range from 30 to 120 dtex.

12. A hook and loop fastener according to any one of claims 1 to 11, **characterized in that** the textured threads are introduced into the bonded nonwoven fabric assembly as warp threads.

13. A hook and loop fastener according to any one of claims 1 to 12, **characterized in that** the number of warp threads (warp count) is in the range from 3 to 25 per 2.54 cm and preferably 20 per 2.54 cm.

14. A hook and loop fastener according to any one of claims 1 to 13, **characterized in that** the stitch count is in the range from 2 to 4 per cm.

15. A method of forming a hook and loop fastener according to any one of claims 1 to 14, **characterized in that** the bonded nonwoven fabric assembly is fed in the stretched state to a Raschel process for introducing the warp threads.

## Revendications

1. Liaison agrippante pour objets plats, en particulier pour produits textiles, avec une partie à crochets et une partie d'accrochage, la partie d'accrochage étant constituée d'un support en composite de feutre, des fils polymères texturés étant incorporés dans le composite de feutre pour les boucles de la partie d'accrochage, **caractérisée en ce que** le composite de feutre est constitué de fibres sans fin non boudées d'une résistance mécanique élevée et est formé d'au moins deux couches de feutre.

2. Liaison agrippante selon la revendication 1, **caractérisée en ce que** le composite de feutre est constitué d'une composition de feutre filé/soufflé à t'état fondu ou d'une composition de feutre filé/soufflé à l'état fondu/feutre filé.

3. Liaison agrippante selon la revendication 2, **caractérisée en ce que** la couche de soufflé à l'état fondu est lissée en film avant l'incorporation des fils.

4. Liaison agrippante selon l'une des revendications 1 à 3, **caractérisée en ce que** le composite de feutre est soudé de manière répartie à la façon d'un motif avant l'incorporation des fils.

5. Liaison agrippante selon t'une des revendications 1 à 4, **caractérisée en ce que** le composite de feutre est calendré sur toute sa surface avant l'incorporation des fils.

6. Liaison agrippante selon l'une des revendications 1 à 5, **caractérisée en ce que** le composite de feutre est doté d'une sous-couche en film.

7. Liaison agrippante selon la revendication 6, **caractérisée en ce que** le film est relié par extrusion à la couche supérieure constituée du composite de feutre.

8. Liaison agrippante selon l'une des revendications 1 à 7, **caractérisée en ce que** le composite de feutre présente un poids par unité de surface de 7 à 70 g/m² et de préférence de 10 à 50 g/m².

9. Liaison agrippante selon l'une des revendications 1 à 8, **caractérisée en ce que** les feutres et/ou les fils texturés sont constitués de filaments identiques ou différents homofils et/ou biconstituants.

10. Liaison agrippante selon l'une des revendications 1 à 9, **caractérisée en ce que** le composite de feutre et les fils texturés sont constitués d'un polymère biodégradable.

11. Liaison agrippante selon l'une des revendications 1 à 10, **caractérisée en ce que** les fils texturés présentent un titre (épaisseur des fils) de 20 à 200 dtex et de préférence de 30 à 120 dtex.

12. Liaison agrippante selon l'une des revendications 1 à 11, **caractérisée en ce que** les fils texturés sont incorporés comme fils de chaîne dans le composite de feutre.

13. Liaison agrippante selon l'une des revendications 1 à 12, **caractérisée en ce que** le nombre des fils de chaîne (densité de chaîne) est de 3 à 25 tous les 2,54 cm et de préférence de 20 tous les 2,54 cm.

14. Liaison agrippante selon l'une des revendications 1 à 13, **caractérisée en ce que** le nombre des mailles est de 2 à 4 par cm.

15. Procédé de fabrication d'une liaison agrippante selon l'une des revendications 1 à 14, **caractérisé en ce que** le composite de feutre est amené sous forme étirée à une opération Raschel pour l'incorporation des fils de chaîne.
